Europäisches Patentamt

European Patent Office

Office européen des brevets

(11) Veröffentlichungsnummer: **0 579 915 A1**

## EUROPÄISCHE PATENTANMELDUNG

(21) Anmeldenummer: **93107492.6**

(22) Anmeldetag: **08.05.93**

(51) Int. Cl.5: **A61K 31/595**, A61K 31/59,
//(A61K31/59,31:20)

(30) Priorität: **20.05.92 CH 1619/92**
**26.03.93 CH 926/93**

(43) Veröffentlichungstag der Anmeldung:
**26.01.94 Patentblatt 94/04**

(84) Benannte Vertragsstaaten:
**AT BE CH DE DK ES FR GB GR IE IT LI LU MC NL PT SE**

(71) Anmelder: **F. HOFFMANN-LA ROCHE AG**
**Grenzacherstrasse 124**
**CH-4002 Basel(CH)**

(72) Erfinder: **Bollag, Werner**
**10 Mythenstrasse**
**CH-4054 Basel(CH)**
Erfinder: **Brockhaus, Manfred**
**29 Talweg**
**CH-4126 Bettingen(CH)**
Erfinder: **Hunziker, Willi**
**84 Margarethenstrasse**
**CH-4053 Basel(CH)**

(74) Vertreter: **Grossner, Lutz, Dr. et al**
**Grenzacher Strasse 124**
**Postfach 3255**
**CH-4002 Basel (CH)**

(54) **Pharmazeutisches Präparat, enthaltend 9-cis- oder 13-cis-Retinsäure oder Acitretin und ein Vitamin-D-Derivat.**

(57) Pharmazeutisches Präparat, enthaltend als Wirkstoffe 9-cis- oder 13-cis- Retinsäure oder Acitretin, ein pharmazeutisch anwendbares Salz oder einen Ester davon, ein Vitamin D-Derivat, und übliche pharmazeutische Trägerstoffe.

EP 0 579 915 A1

Die vorliegende Erfindung betrifft pharmazeutische Präparate enthaltend 9-cis- oder 13-cis-Retinsäure oder Acitretin (all(E)-9-(4-Methoxy-2,3,6-trimethylphenyl)-3,7-dimethyl-2,4,6,8-nonatetraensäure), ein pharmazeutisch anwendbares Salz oder Ester davon, und ein Vitamin D-Derivat. Es wurde gefunden, dass solche Präparate zur Behandlung von Psoriasis, von Osteoporose und von Präcancerosen und Tumoren, sowie zur Behandlung von pathologischen oder unerwünschten Immunreaktionen verwendet werden können. Die Erfindung betrifft somit auch die Verwendung von 9-cis- oder 13-cis-Retinsäure oder Acitretin, pharmazeutisch anwendbaren Salzen oder Estern davon zur kombinierten gleichzeitigen, getrennten oder zeitlich abgestuften Anwendung mit Vitamin D-Derivaten bei der Behandlung der genannten Erkrankungen und Anomalien. Schliesslich betrifft die Erfindung die Verwendung von 9-cis-oder 13-cis-Retinsäure oder Acitretin, pharmazeutisch anwendbaren Salzen oder Estern davon bei der Herstellung pharmazeutischer Präparate zur kombinierten Anwendung mit Vitamin D-Derivaten bei der Behandlung der oben genannten Erkrankungen und Anomalien.

Beispiele pharmazeutisch anwendbarer Salze der 9-cis-bzw. 13-cis-Retinsäure und Acitretin sind Alkalisalze, wie das Na- und K-Salz, Erdalkalimetallsalze wie das Ca- und Mg-Salz; sowie das Ammoniumsalz und Alkylammoniumsalze. Beispiele von Estern sind nieder-Alkylester wie der Methyl- und Aethylester wie Etretinat, und aromatische Ester wie der Benzylester.

Beispiele von erfindungsgemäss verwendbaren Vitamin D-Derivaten sind hydroxylierte Vitamin $D_3$-Derivate wie 1$\alpha$-Hydroxy-Vitamin $D_3$, 1$\alpha$,25-Grn/17.3.93 Dihydroxy-Vitamin $D_3$ (Calcitriol), 1$\alpha$,25,26-Trihydroxy-Vitamin $D_3$, 1$\alpha$,23,25-Trihydroxy-Vitamin $D_3$, 24-Fluoro-1$\alpha$,25-dihydroxy-Vitamin $D_3$, 24,24-Difluoro-1$\alpha$,25-dihydroxy-Vitamin $D_3$, 26,26,26-Trifluoro-1$\alpha$,25-dihydroxy-Vitamin $D_3$, 26,26,26,27,27,27-Hexafluoro-1$\alpha$,25-dihydroxy-Vitamin $D_3$; 1$\alpha$,25-Dihydroxy-22,23-dehydro-Vitamin $D_3$, 26,26,26-Trisdeutero-22,23-dehydro-1$\alpha$,25-dihydroxy-Vitamin $D_3$, 26,26,26,27,27,27-Hexakisdeutero-22,23-dehydro-1$\alpha$,25-dihydroxy-Vitamin $D_3$, 26,26,26-Trifluoro-1$\alpha$,25-dihydroxy-22,23-dehydro-Vitamin $D_3$, 26,26,26,27,27,27-Hexafluoro-1$\alpha$,25-dihydroxy-22,23-dehydro-Vitamin $D_3$, 26,26,26,27,27,27-Hexafluoro-1$\alpha$,25-dihydroxy-23,24-dehydro-Vitamin $D_3$, 1$\alpha$,25-Dihydroxy-Vitamin $D_2$, 26,26,26,27,27,27-Hexakisdeutero-1$\alpha$,25-Vitamin $D_2$, 1$\alpha$,25-Dihydroxy-27-nor-Vitamin $D_2$, 1$\alpha$,25,26-Trihydroxy-22,23-dehydro-Vitamin $D_3$, 1$\alpha$,25,26-Trihydroxy-Vitamin $D_2$, 1$\alpha$,25-Dihydroxy-23,24-didehydro-Vitamin $D_3$, 1$\alpha$,25-Dihydroxy-16, 17-dehydro-Vitamin $D_3$, 1$\alpha$,25-Dihydroxy-16,17;23,24-bisdehydro-Vitamin $D_3$, 1$\alpha$,25-Dihydroxy-16,17-dehydro-23,24-didehydro-Vitamin $D_3$, 26,26,26,27,27,27-Hexafluoro-1$\alpha$,25-dihydroxy-16,17-dehydro-23,24-didehydro-Vitamin $D_3$, 1$\alpha$,26,26,26,27,27,27-Heptafluoro-25-hydroxy-23,24-didehydro-Vitamin $D_3$, 1$\alpha$,25-Dihydroxy-3-deoxy-23,24-didehydro-Vitamin $D_3$ und 25-Hydroxy-23,24-didehydro-Vitamin $D_2$.

Die vorstehend genannten Vitamin-D-Derivate und ihre Herstellung sind bekannt, siehe z.B. die US-Patentschriften 3 993 675, 4 022 768, 4 407 754, 4 421 690, 4 594 432, 4 594 346, 4 612 308, 4 613 594, 4 652 405, 4 749 710, 4 804 502, 4 898 855, 4 906 785, 4 929 609, 5 087 619 und 5 120 722.

Das 26,26,26,27,27,27-Hexafluoro-1$\alpha$,25-dihydroxy-16,17-dehydro-23,24-didehydro-Vitamin $D_3$ wurde noch nicht beschrieben. Es kann wie folgt hergestellt werden:

A. 522 mg [3aS-[3(S*),3a$\alpha$,7$\alpha$,7a$\beta$]]-[[3a,4,5,6,7,7a-Hexahydro-3a-methyl-3-(1-methyl-3-butinyl)-1H-inden-7-yl]oxy]trimethylsilan in 15 ml wasserfreiem Tetrahydrofuran werden auf -75 °C gekühlt, danach werden 1,85 ml 1,6M n-Butyllithium in Hexan tropfenweise im Verlauf von 5 Minuten zugegeben. Das Gemisch wird 30 Minuten bei -75 °C gerührt, danach wird ein Strom von Hexafluoraceton 15 Minuten lang bei -75 °C in das Gemisch eingeleitet. Das Reaktionsgemisch wird dann noch eine weitere Stunde gerührt, danach tropfenweise mit einem Gemisch von 2M KHCO$_3$ und 1M Rochellesalz versetzt. Das Gemisch wird 1 Stunde bei Raumtemperatur gerührt und danach mit 25 ml der gleichen Salzlösung verdünnt. Nach Extraktion mit Methylenchlorid wird die organische Phase mit 50 ml der gleichen Salzlösung gewaschen, über Natriumsulfat getrocknet und eingedampft. Der ölige Rückstand wird mit Benzol azeotrop destilliert und liefert 2,38 g eines öligen Rohproduktes, das durch Flash-Chromatographie (Aethylacetat/Hexan 1:9) gereinigt wurde.

B. 812 mg des in A. erhaltenen [3aS-[3(S*),3a$\alpha$,7$\alpha$:,7a$\beta$]]-1,1,1-Trifluor-6[3a,4,5,6,7,7a-hexahydro-3a-methyl-7-[(trimethylsilyl)oxy]-1H-inden-3-yl]-2-(trifluormethyl)-3-heptin-2-ols in 18 ml wasserfreiem Tetrahydrofuran werden mit 5,34 ml Tetrabutylammoniumfluorid in Tetrahydrofuran versetzt. Das Gemisch wird unter Argon 80 Minuten bei Raumtemperatur gerührt. Danach wird die Reaktion durch Zusatz von 9 ml halbgesättigter NaHCO$_3$-Lösung gestoppt und das Gemisch wird weitere 20 Minuten bei Raumtemperatur gerührt. Das überschüssige Tetrahydrofuran wird abgedampft und es werden zusätzlich 9 ml Bicarbonat zugesetzt. Das Gemisch wird mit Aethylacetat extrahiert. der Extrakt mit Kochsalzlösung gewaschen, über Natriumsulfat getrocknet und eingedampft. Das Rohprodukt wird durch Flash-Chromatographie (Aethylacetat/Hexan 1:2) gereinigt.

C. 100 mg des in Absatz B. erhaltenen [3aR,-[1(R*),3a$\alpha$:,4$\beta$,7a$\beta$]]-3,3a,5,6,7,7a-Hexahydro-7a-methyl-1-[6,6,6-trifluor-5-hydroxy-1-methyl-5-(trifluormethyl)-3-hexinyl]-4H-inden-4-ols in 6 ml wasserfreiem Methylenchlorid werden bei Raumtemperatur in mehreren Anteilen mit 176 mg Pyridiniumchlorochromat versetzt. Das Gemisch wird 50 Minuten bei Raumtemperatur unter Argon gerührt. Danach werden 9 ml Aether zugesetzt und es wird 10 Minuten weiter gerührt, danach filtriert und das Filtrat zur Trockene eingedampft. Das Rohprodukt wird an Silicagel mit Aethylacetat/Hexan 1:3) chromatographiert und liefert einen amorphen Feststoff.

D. 333 mg [3S-(3α,5ß,Z)]-2-[2-[2-Methylen-3,5-bis[[(1,1-dimethyläthyl)-dimethylsilyl]oxy]cyclohexyliden]-äthyl]diphenylphosphinoxid in 7 ml wasserfreiem Tetrahydrofuran werden bei -75°C mit 0,325 ml 1,6M n-Butyllithium in Hexan unter Argon tropfenweise versetzt, bis das Reaktionsgemisch eine rote Farbe entwickelte. Nach 6 Minuten Rühren wird eine Lösung von 73 mg [3aR-[1(R*),3aα,7aß]]-3,3a,5,6,7,7a-Hexahydro-7a-methyl-1-[6,6,6-trifluor-5-hydroxymethyl-5-(trifluormethyl)-3-hexinyl]-4H-inden-4-on (Produkt von Absatz C.) in 5 ml wasserfreiem Tetrahydrofuran im Verlauf von 15 Minuten zugesetzt. Das Reaktionsgemisch wird 1 Stunde im Dunkeln bei -75°C gerührt, danach wird die Reaktion durch Zusatz von 2,6 ml eines 1:1-Gemisches von 2N Rochellesalz und 2N $KHCO_3$-Lösung gestoppt und auf Zimmertemperatur aufgewärmt. Man verdünnt das Reaktionsgemisch dann mit 10 ml der gleichen Salzlösung und extrahiert mit Aethylacetat. Der Extrakt wird mit Kochsalzlösung gewaschen, über Natriumsulfat getrocknet und eingedampft. Das Rohprodukt wird durch Flash-Chromatographie an Silicagel mit Aethylacetat/Hexan 1:5) gereinigt und lieferte die gewünschte Verbindung, die noch Silylschutzgruppen enthält.

E. 92 mg der in Abschnitt D. erhaltenen silylierten Verbindung in 5 ml wasserfreiem Tetrahydrofuran werden in einem dunkelwandigen Reaktionsgefäss mit 0,89 ml 1M Tetrabutylammoniumfluorid in Tetrahydrofuran versetzt. Das Gemisch wurde 16 Stunden unter Argon gerührt. Danach werden 3 ml halbgesättigter $NaHCO_3$-Lösung zugesetzt. Man rührt 20 Minuten bei Raumtemperatur, danach wird mit Aethylacetat extrahiert, der Extrakt mit halbgesättigter $NaHCO_3$-Lösung und Kochsalzlösung gewaschen, über Natriumcarbonat getrocknet und eingedampft. Das Rohprodukt wird durch Flash-Chromatographie mit Aethylacetat/Hexan 4:1) gereinigt und liefert die Verbindung als schaumiges Glas, $[\alpha]_D^{25} = +59,1°$ (c = 0,11, Methanol).

Erfindungsgemäss kann die 9-cis- oder 13-cis-Retinsäure oder Acitretin bzw. deren Salze oder Ester in Form pharmazeutischer Präparate angewandt werden, die auch ein Vitamin D-Derivat enthalten, oder als ad hoc-Kombination mit Vitamin D-Derivate enthaltenden Präparaten angewandt werden.

Bevorzugt ist die Verwendung von 9-cis-Retinsäure, pharmazeutisch anwendbaren Salzen oder Estern davon und Vitamin D-Derivaten, insbesondere 9-cis-Retinsäure und Calcitriol.

Zur Behandlung der Psoriasis können die Wirkstoffe topisch oder oral verabreicht werden. Topische Präparate können als Crèmes, Salben, Lotionen, Tinkturen oder Gele vorliegen, welche die Wirkstoffe zusammen mit in solchen Präparaten üblichen Trägerstoffen enthalten. Der Gehalt an 9-cis- oder 13-cis-Retinsäure oder Acitretin, Salzen oder Estern davon in diesen Präparaten kann etwa 0,001-0,1 Gew.-%, vorzugsweise 0,003-0,03 Gew.-% betragen. Der Gehalt an Vitamin D-Derivat in solchen Präparaten kann etwa 1 μg/g bis etwa 100 μg/g betragen. Diese Präparate werden auf die erkrankten Hautstellen entsprechend den Bedürfnissen des Patienten, z.B. ein- bis zweimal täglich, aufgetragen.

Präparate zur oralen Verabreichung können in Form von Tabletten, Kapseln, Lösungen oder Emulsionen vorliegen. Zur Behandlung der Psoriasis können solche Präparate in Dosierungen von etwa 0,01 mg bis etwa 3 mg 9-cis-oder 13-cis-Retinsäure oder Acitretin bzw. Salze oder Ester davon pro kg Körpergewicht pro Tag, vorzugsweise etwa 0,025 mg/kg bis etwa 1,5 mg/kg täglich; und etwa 0,001 μg/kg bis etwa 0,1 μg/kg Vitamin D-Derivat, vorzugsweise etwa 0,005 μg/kg bis etwa 0,05 μg/kg täglich verabreicht werden. Feste Darreichungsformen wie Tabletten und Kapseln enthalten pro Dosiseinheit zweckmässig etwa 1 mg bis etwa 50 mg 9-cis- oder 13-cis-Retinsäure Salze oder Ester davon bzw. etwa 0,1 μg bis etwa 1 μg Vitamin D-Derivat.

Zur Behandlung und Prävention von Tumoren können die Wirkstoffe bzw. die erfindungsgemässen Präparate enteral, parenteral oder topisch verabreicht werden. Beispiele von Tumoren, die mit den erfindungsgemässen Präparaten bzw. der erfindungsgemässen Wirkstoffkombination behandelt werden können, sind hämatologische Tumoren, wie Leukämien, insbesondere akute promyelocytäre Leukämie und Lymphämie. Weiterhin können präcanceröse Läsionen des Epithelialgewebes, z.B. aktinische Keratosen der Haut, orale Leukoplakien, Larynxdysplasien Bronchialdysplasien, und Cervikaldysplasien, sowie Karzinome von Haut, Mundhöhle, Pharynx, Larynx, Bronchien, Magen, Colon, Harnblase, Gebärmutter, Mamma und Prostata durch Verabreichung einer wirksamen Menge der erfindungsgemässen Präparate bzw. Wirkstoffkombination behandelt werden.

Beispiele pathologischer oder unerwünschten Immunreaktionen sind Autoimmunerkrankungen wie rheumatoide Arthritis, multiple Sklerose, Insulin-abhängiger Diabetes, Lupus erythematosus, Pemphigus vulgaris, Pemphigus foliaceus, Myasthenia gravis, ankylosierende Spondylitis, Autoimmunerkrankungen der Schildrüse wie Morbus Hashimoto und primäres Schilddrüsenversagen; Skleroderma, Uveitis, Morbus Behcet, Morbus Crohn, autoimmunbedingte Myocarditis und autoimmunbedingte Polyglandulärsyndrome; weiterhin Allergien wie allergische Rhinitis, atopische Dermatitis, Asthma und Zöliakie. Weitere Indikationen sind unerwünschte Immunreaktionen bei Organ- oder Zelltransplantationen, wie Nieren-, Herz-, Beta-Zellinsel der Bauchspeicheldrüse, Knochenmark- und Lebertransplantationen.

Ein weiterer Aspekt der Erfindung betrifft die Verwendung der erfindungsgemässen Präparate bzw. Wirkstoffkombination zur vorzugsweise enteralen oder parenteralen Behandlung und Prävention der Osteoporose. Bei allen diesen Indikationen können die Wirkstoffe in den oben angegebenen Dosierungsbereichen angewandt werden, wobei die individuelle Dosierung von der Alt der zu behandelnden Erkrankung sowie vom Alter und Zustand des Patienten abhängt und im Rahmen des ärztlichen Fachwissens bestimmt werden kann. Die Erfindung wird durch die nachstehenden Beispiele weiter erläutert.

Beispiel 1

| Kapsel mit 9-cis-Retinsäure | |
|---|---|
| 9-cis-Retinsäure | 20,0 mg |
| Gelatine (Bloomzahl 30) | 70,0 mg |
| Maltodextrin | 108,0 mg |
| dl-$\alpha$-Tocopherol | 2,0 mg |
| Na-Ascorbat | 10,0 mg |
| mikrokristalline Cellulose | 48,0 mg |
| Mg-Stearat | 2,0 mg |
| Total | 260 mg |

Der Wirkstoff wird in einer Lösung von Gelatine, Maltodextrin, Tocopherol und Na-Ascorbat feucht gemahlen und die erhaltene Suspension sprühgetrocknet. Danach werden die Cellulose und das Mg-Stearat zugemischt und je 260 mg des Gemischs werden in Hartgelatinekapseln abgefüllt.

Beispiel 2

| Kapsel mit Calcitriol | |
|---|---|
| Calcitriol | 0,25 $\mu$g |
| butyliertes Hydroxytoluol | 0,016 mg |
| buyliertes Hydroxyanisol | 0,016 mg |
| fraktioniertes Kokosöl | ad 160,0 mg |

Die Bestandteile werden gemischt und die ölige Lösung wird unter Inertgas in Weichgelatinekapseln mit je 160 mg Inhalt abgefüllt.

Beispiel 3

| Kapsel mit 9-cis-Retinsäure und Calcitriol | |
|---|---|
| Calcitriol | 0,25 $\mu$g |
| 9-cis-Retinsäure | 20 mg |
| Polyäthylenglykol 400 | 200 mg |
| butyliertes Hydroxyanisol | 0,1 mg |

Die Bestandteile werden gemischt und unter Inertgas in Weichgelatinekapseln mit einem Füllgewicht von 220 mg abgefüllt.

Beispiel 4

| Crème mit 9-cis-Retinsäure und Calcitriol | |
| --- | --- |
| Calcitriol | 2 mg |
| 9-cis-Retinsäure | 30 mg |
| Cetylalkohol | 1,5 mg |
| Stearylalkohol | 2,5 mg |
| Sorbitanmonostearat | 2,0 mg |
| Glycerylmonostearat und Polyoxyäthylenglykolstearat | 4,0 mg |
| Polysorbat 60 | 1,0 mg |
| Mineralöl | 4,0 mg |
| Propylenglykol | 5,0 mg |
| Propylparaben | 0,05 mg |
| butyliertes Hydroxyanisol | 0,05 mg |
| Sorbitlösung | 2,0 mg |
| Na-EDTA | 0,01mg |
| Methylparaben | 0,18 mg |
| Dest. Wasser | q.s. ad 100 g |

Beispiel 5

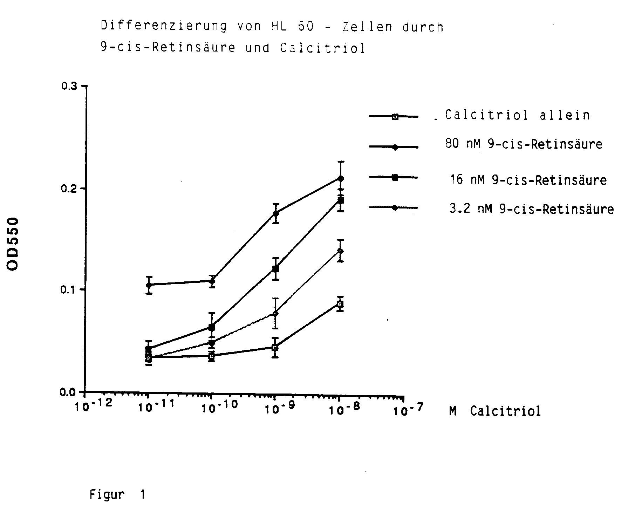

Die Wirkung der erfindungsgemässen Kombination von 9-cis-Retinsäure und Calcitriol auf die Differenzierung menschlicher promyelocytischer Leukämiezellen (HL-60) kann in vitro in der in Cancer Research 45, 4244 (1985) beschriebenen Versuchsanordnung gezeigt werden. Die mit verschiedenen Konzentrationen der Wirkstoffe erhaltenen Effekte auf die Zelldifferenzierung (gemessen durch Nachweis der Reduktionswirkung auf Nitroblau-Tetrazolium, NBT) kann der Figur 1 entnommen werden. Die Messung der NBT-Reduktion erfolgte nach einer von Pick et al. in J. Reticul. Soc. 30, 581 (1981) beschriebenen, modifizierten Methode. Jeweils $3 \cdot 10^4$ Zellen in 200 $\mu$l wurden mit den Wirkstoffen 48 Stunden inkubiert. Die Zellen wurden abzentrifugiert und mit jeweils 100 $\mu$l vorgewärmter NBT-Lösung (1 mg/ml, verdünnt mit Dulbeccos PBS) und PMA (123 mg/ml in DMSO) versetzt und 1 Stunde bei 37°C inkubiert. Nach Zentrifugieren wurden 100 $\mu$l 90% DMF, verdünnt mit 10% SDS zugesetzt, bei 37°C inkubiert und die Extinktion (OD) bei 550 nm gemessen.

Die Kurven in Fig. 1 zeigen den Effekt von Calcitriol allein, sowie von Kombinationen von wechselnden Mengen Calcitriol mit (von oben nach unten) 80 nM, 16 nM und 3,2 nM 9-cis-Retinsäure.

Beispiel 6

Patienten mit multiplen aktinischen Keratosen wurden topisch mit 9-cis-Retinsäure und Calcitriol behandelt. 9-cis-Retinsäure wurde als 0,01%ige Lösung in Aethanol/Propylenglykol (50:50), Calcitriol als 0,0025%ige Crème angewandt. Die Behandlung erfolgte während 4-16 Wochen, wobei die Präparate einmal täglich auf die Haut appliziert wurden. Dabei wurde zuerst 9-cis-Retinsäure-Lösung appliziert, nach Trocknen (3 Minuten) Calcitriol-Crème. Ein Okklusiv-Verband wurde nicht verwendet.

Die Behandlung verschiedener Patientengruppen ergab die folgenden Resultate:

A. Patientengruppe: 16 Patienten

Behandlungsdauer: 4 Wochen

Behandlungserfolg:

6 Patienten: leichte Besserung

5 Patienten: mässige Besserung

5 Patienten: deutliche Besserung

B. Patientengruppe: 16 Patienten

Behandlungsdauer: 8 Wochen

Behandlungserfolg:

3 Patienten: leichte Besserung

4 Patienten: mässige Besserung

9 Patienten: deutliche Besserung

C. Patientengruppe: 7 Patienten
Behandlungsdauer: 12 Wochen
Behandlungserfolg:
Bei allen 7 Patienten: deutliche Besserung
D. Patientengruppe: 3 Patienten
Behandlungsdauer: 16 Wochen
Behandlungserfolg:
Bei allen 3 Patienten: deutliche Besserung
In allen Fällen trat ein leichtes Erythem, aber keine störenden Symptome (Brennen, Jucken) auf.

**Patentansprüche**

1. Pharmazeutisches Präparat, enthaltend als Wirkstoffe 9-cis- oder 13-cis-Retinsäure oder Acitretin, ein pharmazeutisch anwendbares Salz oder einen Ester davon, ein Vitamin D-Derivat, und übliche pharmazeutische Trägerstoffe.

2. Pharmazeutisches Präparat, enthaltend als Wirkstoffe 9-cis- Retinsäure, ein pharmazeutisch anwendbares Salz oder einen Ester davon, ein Vitamin D-Derivat, und übliche pharmazeutische Trägerstoffe.

3. Präparat nach Anspruch 2, wobei die Wirkstoffe 9-cis-Retinsäure und Calcitriol sind.

4. Erzeugnisse, enthaltend 9-cis- oder 13-cis-Retinsäure oder Acitretin, ein pharmazeutisch anwendbares Salz oder einen Ester davon, und ein Vitamin D - Derivat als Kombinationspräparat zur gleichzeitigen, getrennten oder zeitlich abgestuften Anwendung bei der Behandlung von Psoriasis, Osteoporose oder Tumoren.

5. Erzeugnisse gemäss Anspruch 4 zur Anwendung bei der Behandlung von pathologischen oder unerwünschten Immunreaktionen.

6. Erzeugnisse gemäss Anspruch 4 oder 5 enthaltend 9-cis-Retinsäure und Calcitriol.

7. Handelspackung, enthaltend als pharmazeutischen Wirkstoff 9-cis- oder 13-cis-Retinsäure oder Acitretin, ein pharmazeutisch anwendbares Salz oder einen Ester davon, zusammen mit Instruktionen für deren Verwendung in Kombination mit einem Vitamin D-Derivat zur gleichzeitigen, getrennten oder zeitlich abgestuften Anwendung bei der Behandlung von Psoriasis, Osteoporose oder Tumoren.

8. Handelspackung gemäss Anspruch 7 zur Anwendung bei der Behandlung von pathologischen oder unerwünschten Immunreaktionen.

9. Handelspackung gemäss Anspruch 7 oder 8 enthaltend 9-cis-Retinsäure zusammen mit Instruktionen zur kombinierten Anwendung mit Calcitriol.

10. 9-cis- oder 13-cis-Retinsäure und Acitretin, pharmazeutisch anwendbare Salze oder Ester davon in Kombination mit einem Vitamin D - Derivat zur Verwendung als Arzneimittel.

11. 9-cis-Retinsäure und Calcitriol zur Verwendung gemäss Anspruch 10.

12. Verwendung von 9-cis- oder 13-cis-Retinsäure oder Acitretin, pharmazeutisch anwendbaren Salzen oder Estern davon bei der Herstellung von pharmazeutischen Präparaten zur kombinierten Anwendung mit einem Vitamin - D - Derivat bei der Behandlung von Psoriasis, Osteoporose oder Tumoren.

13. Verwendung gemäss Anspruch 12 bei der Behandlung von pathologischen oder unerwünschten Immunreaktionen.

14. Verwendung von 9-cis- Retinsäure zur kombinierten Anwendung mit Calcitriol gemäss Anspruch 12 oder 13.

Differenzierung von HL 60 - Zellen durch
9-cis-Retinsäure und Calcitriol

Figur 1

Europäisches
Patentamt

**EUROPÄISCHER TEILRECHERCHENBERICHT** Nummer der Anmeldung

der nach Regel 45 des Europäischen Patentübereinkommens für das weitere Verfahren als
europäischer Recherchenbericht gilt

EP   93 10 7492
Seite 1

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl. 5) |
|---|---|---|---|
| X | STN INTERNATIONAL, KARLSRUHE. FILE CA, CHEMICAL ABSTRACTS.<br><br>AN=CA118(5):32608a. Y. XIE, 'Effects of 1.alpha.,25-dihydroxyvitamin D3 and 13-cis-retinoic acid on the induction of differentiation of isolated acute non-lymphoblastic leukemia cells'<br>* Zusammenfassung *<br>& ZHONGHUA XUEYEXUE ZAZHI, 13(3), 125-8, 1992<br>--- | 1,4,5,7, 8,10,12, 13 | A61K31/595 //(A61K31/59, 31:20) |
| X | STN INTERNATIONAL, KARLSRUHE. FILE CA, CHEMICAL ABSTRACTS.<br><br>AN=CA115(25):270136c. T. CHENG, 'Differentiation of human leukemic cells induced by retinoic acid in combination with 1,25-dihydroxyvitamin D3'.<br>* Zusammenfassung *<br>& SHANGHAI YIXUE, 14(5), 280-3, 1991.<br>---<br><br>-/-- | 1,4,5,7, 8,10,12, 13 | |

**RECHERCHIERTE SACHGEBIETE (Int. Cl. 5)**

A61K

## UNVOLLSTÄNDIGE RECHERCHE

Nach Auffassung der Recherchenabteilung entspricht die vorliegende europäische Patentanmeldung den Vorschriften des Europäischen patentübereinkommens so wenig, daß es nicht möglich
ist, auf der Grundlage einiger Patentansprüche sinnvolle Ermittlungen über den Stand der
Technik durchzuführen.
Vollständig recherchierte Patentansprüche:
Unvollständig recherchierte Patentansprüche:
Nicht recherchierte Patentansprüche:
Grund für die Beschränkung der Recherche:


Siehe Ergänzungsblatt C

| Recherchenort | Abschlußdatum der Recherche | Prüfer |
|---|---|---|
| DEN HAAG | 15 SEPTEMBER 1993 | ORVIZ DIAZ P. |

KATEGORIE DER GENANNTEN DOKUMENTEN

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus andern Gründen angeführtes Dokument
............................................................
& : Mitglied der gleichen Patentfamilie, übereinstimmendes
   Dokument

Europäisches
Patentamt

**EUROPÄISCHER TEILRECHERCHENBERICHT**

Nummer der Anmeldung

EP    93 10 7492
Seite 2

| EINSCHLÄGIGE DOKUMENTE | | | KLASSIFIKATION DER ANMELDUNG (Int. Cl. 5) |
|---|---|---|---|
| Kategorie | Kennzeichnung des Dokuments mit Angabe, soweit erforderlich der maßgeblichen Teile | Betrifft Anspruch | |
| X | STN INTERNATIONAL, KARLSRUHE. FILE CA, CHEMICAL ABSTRACTS. AN=CA112(10):84170x. Y. KAWAJIRI, 'Pharmaceuticals in combination with Cuscuta chinensis seed extracts for treatment of acne'. * Zusammenfassung * & JP-A-63 060 910 (SHISEIDO CO., LTD.) 17. März 1988 --- | 1,4,5,7, 8,10,13 | |
| X,Y | MOL. CELL. ENDOCRINOL. Bd. 76, Nr. 1-3, 1991, Seiten 149 - 159 A. PIRSKANEN 'Modulation of 1,25(OH)2D3-induced osteocalcin synthesis in human osteosarcoma cells by other steroidal hormones' * das ganze Dokument * --- | 1-14 | **RECHERCHIERTE SACHGEBIETE (Int. Cl. 5)** |
| X,Y | J. STEROID. BIOCHEM. MOL. BIOL. Bd. 39, Nr. 4A, 1991, Seiten 455 - 460 M. KOGA 'Retinoic acid acts synergistically with 1,25-dihydroxyvitamin D3 or antioestrogen to inhibit T-47D human breast cancer cell proliferation.' * das ganze Dokument * --- | 1-14 | |
| X,Y | LEUK. RES. Bd. 15, Nr. 12, 1991, Seiten 1145 - 1152 M. TAIMI 'Synergistic effect of retinoic acid and 1,25-dihydroxyvitamin D3 on the differentiation of the human monocytic cell line U937' * das ganze Dokument * --- | 1-14 | |
| Y | EUR. J. CLIN. INVEST. Bd. 16, Nr. 4, 1986, Seiten 297 - 301 A. NAGLER 'Differentiation of bone marrow cells from myelodysplastic patients in the presence of 1,25 dihydroxyvitamin D3 or 13-cis retinoic acid.' * das ganze Dokument * --- | 1-14 | |

-/--

Europäisches
Patentamt

EUROPÄISCHER TEILRECHERCHENBERICHT

Nummer der Anmeldung

EP    93 10 7492
Seite 3

## EINSCHLÄGIGE DOKUMENTE

KLASSIFIKATION DER
ANMELDUNG (Int. Cl. 5)

| Kategorie | Kennzeichnung des Dokuments mit Angabe, soweit erforderlich der maßgeblichen Teile | Betrifft Anspruch |
|---|---|---|
| Y | J. MED. COLL. PLA<br>Bd. 5, Nr. 3, 1990,<br>Seiten 282 - 286<br>C. TAO 'Inhibition of proliferation of human megakaryoblastic leukemic cells by 1,25-dihydroxyvitamin D3 and retinoic acid'<br>* das ganze Dokument *<br>--- | 1-14 |
| Y | NATURE<br>Bd. 355, Nr. 6358, 23. Januar 1992,<br>Seiten 359 - 361<br>A.A. LEVIN '9-Cis retinoic acid stereoisomer binds and activates the nuclear receptor RXRalpha.'<br>* das ganze Dokument *<br>--- | 2,3,6,9, 11,14 |
| Y | CELL<br>Bd. 68, Nr. 2, 24. Januar 1992,<br>Seiten 397 - 406<br>R.A. HEYMAN '9-Cis retinoic acid is a high affinity ligand for the retinoid X receptor'<br>* das ganze Dokument *<br>----- | 2,3,6,9, 11,14 |

RECHERCHIERTE
SACHGEBIETE (Int. Cl. 5)

EPO FORM 1503 03.82 (P04E12)

EP 93 10 7492

-C-

UNVOLLSTÄNDIGE RECHERCHE

Unvollständig recherchierte Patentansprüche:  1,2,4,5,7,8,10,12,13

Sinnvolle Ermittlungen nicht möglich

Unklarheiten, Zusammenhanglosigkeit, Widersprüche....

Der Ausdruck "Vitamin D-Derivat" beinhaltet eine
grosse Zahl von Verbindungen.
Durch derartige Ausdrücke werden spezifische chemische
Substanzen nicht eindeutig charakterisiert.

Die Recherche musste auf das generelle Konzept, sowie
auf die in den Ansprüchen und in den pharmakologischen
Beispielen genannten Substanzen beschränkt werden.
(Richtlinien für die Prüfung im Europäischen Patentamt,
Teil B, Kapitel II.7., letzter Satz und Kapitel III.3.7).